# EUROPEAN PATENT SPECIFICATION

(11) **EP 1 443 983 B1**
(45) Date of publication and mention of the grant of the patent: **26.10.2005**
(21) Application number: 02786318.2
(22) Date of filing: 12.11.2002
(51) Int. Cl.: A61L 31/04, C12N 5/08

(54) **NERVE REPAIR UNIT AND METHOD OF PRODUCING IT**
NERVENREPARATURGERÄT UND VERFAHREN ZU SEINER HERSTELLUNG
UNITE DE REPARATION DES NERFS ET SON PROCEDE DE PRODUCTION

(30) Priority: 16.11.2001 SE 0103827
(43) Date of publication of application: 11.08.2004
(73) Proprietor: Wiberg, Mikael, 870 33 Docksta (SE)
(72) Inventor: Wiberg, Mikael, 870 33 Docksta (SE)
(74) Representative: Onn, Thorsten
(86) International application number: PCT/SE2002/002058
(87) International publication number: WO 2003/041758

(56) References cited:
- AFSHIN MOSAHEBI ET AL: 'Effect of allogenic Schwann cell transplantation on peripheral nerve regeneration' EXPERIMENTAL NEUROLOGY vol. 173, 2002, pages 213 - 223, XP002977354
- AFSHIN MOSAHEBI ET AL: 'Retroviral labeling of Schwann cells: In vitro characterization and in vivo transplantation to improve peripheral nerve regeneration' GLIA vol. 34, 2001, pages 8 - 17, XP002977359
- AFSHIN MOSEHABI ET AL: 'A novel use of alginate hydrogel as Schwann cell matrix' TISSUE ENGINEERING vol. 7, no. 5, 2001, pages 525 - 534, XP002977356
- EVANS G.R.D. ET AL: 'In vivo evaluation of poly(L-lactic acid) porous conduits for peripheral nerve regeneration' BIOMATERIALS vol. 20, 1999, pages 1109 - 1115, XP002977355
- HAZARI A. ET AL: 'A resorbale nerve conduit as an alternative to nerve autograft in nerve gap repair' BRITISH JOURNAL OF PLASTIC SURGERY vol. 52, 1999, pages 653 - 657, XP002977372

## Description

The present invention relates to a nerve repair unit and a method of producing it. The nerve repair unit comprises a resorbable polymeric support and an alginate matrix containing human Schwann cells.

### BACKGROUND

It is well known that neural cells undergo changes after nerve injury, and some cells die. Further, it is known today that this cell death is extensive and results in changes of the projection pattern that the injured nerve has on the spinal cord level. Despite optimal microsurgical repair immediately after an induced experimental animal nerve injury a 25 -50 % loss of nerve cells arises, and is accompanied by an even greater loss of sensory reflex contacts inside the spinal cord (1-8). This nerve cell death is initiated within the first few days after the nerve injury and continues for several months (9). This cell death contributes to the impaired recovery of sensation in patients with nerve injuries, since a loss of nerve cells makes it more difficult for outgrowth of sensory nerves to the target area (10).

For reduction of this cell death, groups of small proteins (growth factors) are used, which are of great importance for the nerve cell function and survival. At nerve injury, these factors are released from cells, so-called Swann cells, that surround the injured nerve ends. However, due to leakage to the surrounding tissue, the concentration does not become high enough to prevent the nerve cell death. By introducing these factors into the spinal cord canal during the nerve healing, it has been demonstrated on rat that they can completely eliminate the nerve cell death (11).

In clinical practice most patients with major proximal nerve injuries (so-called plexus injuries) after difficult childbirth and traffic accidents are subjected to surgery after 4-12 weeks, since this time is necessary for evaluating the extent of the nerve injuries.

It would be desirable, especially in these emergency cases, when the patients come to the hospital, to take a microscopic nerve biopsy for cultivation of the patient's own Schwann cells. At the time for reconstruction of the injured area, it would be desirable to have means for substituting lost nerve tissue with the patient's own Schwann cells.

### DESCRIPTION OF THE INVENTION

The present invention provides means for repair of injured human nerves. Thus, the invention is directed to a nerve repair unit comprising a resorbable polymeric support and an alginate matrix containing human Schwann cells.

The resorbable polymeric support may be any commercially available support or support described in the literature suitable for implantation, especially a resorbable conduit for nerve regeneration.

In an embodiment of the invention the polymer of the resorbable polymeric support is selected from the group consisting of polyhydroxybutyric acid, polyglycolic acid and polylactic acid. In a presently preferred embodiment the polymer is polyhydroxybutyric acid (PHB).

In a presently most preferred embodiment the resorbable polymeric support is a polyhydroxybutyric acid conduit.

The choice of alginate as the matrix to be used in the present invention was preceded by experimental work evaluation also Collagen Type I (Sigma: C7661); Fibrin glue (Tisseel™, Immuno); Hyaluronic acid (Hylan G-F 20, Biomatrix) and Matrigel®: growth factor reduced Matrigel (Collaborative Biomedical Products, Becton Dickinson Labware). They were all inferior to alginate for the purpose of the present invention.

In an embodiment of the invention the alginate matrix is an ultrapure, low viscosity mannuronic acid alginate.

The human Schwann cells may be received from a culture of a biopsy sample from e.g. a branch of the sural nerve of the leg of a donor. Preferably the donor is the patient who is to receive the nerve repair unit of the invention. In case the patient has an open injury; a nerve biopsy sample may be taken from the damaged peripheral nerve for culturing of the Schwann cells.

Another aspect of the invention is directed to a method of producing a nerve repair unit comprising a resorbable polymeric support and an alginate matrix containing human Schwann cells. The method comprises the steps of mixing an alginate in isotonic saline solution with a human Schwann cell suspension in fibronectin, supplying the mixture to the resorbable polymeric support, setting the alginate matrix with a solution of calcium chloride in human cell culture medium, rinsing with the human cell culture medium and keeping the nerve repair unit in the human cell culture medium until use.

In an embodiment of the method of the invention the polymer of the resorbable polymeric support is selected from the group consisting of polyhydroxybutyric acid, polyglycolic acid and polylactic acid, the alginate is ultrapure, low viscosity mannuronic acid alginate and the human cell culture medium is Dublbecco's Minimum Eagles Medium plus Glutamax® (DMEM).

In another embodiment of the method of the invention the human Schwann cells are produced by transporting a sample of a peripheral nerve of a human patient in a transfer medium at ambient temperature within 24 hours to a competent laboratory, upon arrival washing the sample with a human culture medium, removing the perineurum, dividing the nerve branches into fascicles and cutting them into pieces, washing the pieces with a human culture medium and placing them into a culture flask coated with both poly-D-lysine and human laminin and covering them with a Schwann cell culture medium, followed by incubation at 37°C for up to 10 days, changing the medium every 2 days, and removing the nerve segments for digestion in a container with cell culture medium plus collagenase I and dispase I until the segments have broken down, followed by trituration, filtration, washing and centrifugation, resuspending the resulting pellet in transfer medium and plating the suspension on a culture flask coated with both poly-D-lysine and human laminin and after 24 hours changing the medium to a Schwann cell culture medium, followed by cultivation for at least 7 days under conditions removing possible fibroblast contamination and changes of culture medium until the Schwann cells are confluent and ready to split, aspirating the medium and suspending the cells in a trypsinized solvent, followed by centrifugation and washing of the pellet, counting the Schwann cells with a haematocytometer and plating them on a culture flask coated with both poly-D-lysine and human laminin and covering them with a Schwann cell culture medium at a density of 5x 10⁵ cells/ 25 cm², culturing the cells for at least 14 days under change of medium every day to produce an appropriate cell number for transplantation to the patient.

In the experimental part of this description it is shown, in an animal model, that allogenic cells have a survival time that is shorter compared to syngenic cells *in vivo* when no immunosuppression is used. However, allogenic Schwann cells have a survival time, prior to rejection, that may be sufficient for minor nerve injuries with shorter defects in the nerve tissue.

The invention will now be illustrated by description of experiments, but it should be understood that the scope of protection is not limited to specifically mentioned details.

### EXPERIMENTS

### Materials used in the experiments

| **Resorbable polymer** | |
|---|---|
| Poly hydroxy butyrate (PHB) sheets | Astra-tech, Sweden |

| **Matrix** | |
|---|---|
| Alginate, low viscosity mannuronate (LVM) Batch no: 210-241-02 | Pronova Biomedical, Norway |
| Calcium chloride | Sigma |
| Fibronectin, 0.1%, bovine plasma | Sigma |

| **Tissue culture** | |
|---|---|
| Cell growth medium | DMEM plus: 10% fetal calf serum and Penicillin/Streptomycin (100iu-100µg) |
| Collagenase I | Worthington Biochemicals, Code; CLS-I, 125 U/ mg. 1% working solution made with DMEM and aliquoted in 300 µl and kept at -20°C. |
| Complement, rabbit anti mouse | Cedarlane Labs, Cat No: CL3051, 2 vials reconstituted at a time, kept on ice at all times, 1ml sterile water added to each vial, filtered through 0.45µM Filter, stored in liquid Nitrogen as 250 µl aliquots. |
| Cytosine-β-D-arabinofuranoside 5'-tri-Phosphate (Arac-C) | Sigma, Cat No: C3639/C8779, Stock solution ~1mM: 2 mg dissolved in 4 ml PBS, filtered 0.2 µm and stored at -20 °C, 250 µl aliquots |
| Dispase I: | Roche, Cat No: 210 455, 5 mg. Stock solution: 1 ml of sterile water added, stored at -20 °C as 0.1 ml aliquots, i.e. each aliquot ~ 3 U. |
| DMEM with HEPES | Gibco, Cat No: 22320 |
| DMEM, high glucose (4.5 g/l glucose) | Gibco, Cat No |
| Dulbecco's Modified Eagle's Medium plus Glutamax (DMEM) | Gibco, Cat No: 21885 |
| Fetal Calf Serum (FCS) | Imperial Laboratories, 500ml, Batch No: 53265, Heat deactivated at 56°C for 35 minutes, stored at -20°C |
| Forskolin | Calbiochem, Cat No: 344270, 5 mM stock solution made by dissolving 2.0525 mg/ ml in DMSO. Stored at -20°C 50/ 100 µl aliquots |
| BMX, Isobutyl-1-Methylxanthine | Sigma, Cat No: I-5879, Stock solution 50 mM: FW: 222.2, 100 mg dissolved in DMSO. Stored at -20°C as 200 µl aliquots. |
| Insulin, Bovine Pancreas, culture tested | Sigma, Cat No: I 1882, Lot: 57H4626, Stock solution 10 mg/ ml; 10 ml of acidified Water (150 µl of glacial acetic acid added to 15 ml of distilled water, in fume cupboard, 0.2 µm filtered). Dissolved slowly re-filtered with 0.8/0.2 µm. Stored at 4 °C as I ml aliquots. |
| Mouse Anti Human Thy 1.1 | Serotec, Cat NO: MCAP90, 2ml, IgG1 |
| Mouse Anti Mouse/Rat Thy 1.1 | Serotec, Cat No: MCA04G, 0.25mg/ 0.25ml, IgM |
| Penicillin/Streptomycin | Gibco, Cat: 15070-022, Working concentration: 100 iu-100 µg, by adding 2ml to 100 ml of DMEM. |
| Poly-D-Lysine, Lyophilised, MW: 30,000-70,000. | Sigma, Cat No: P7280 |
| Schwann cell growth medium (human) | Rat Schwann cell growth medium (made with high, 4.5g/l, glucose DMEM plus 0.5 mM IBMX, 1.1 ml of stock and 2.5 µg/ml insulin, 28 µl for 100ml. |
| Schwann cell growth medium (neonatal rat) | Cell growth medium plus forskolin, 5µM, GGF 126 ng/ml (of Batch rhGGF2 121195): To 100 ml of cell growth medium 112 µl of froskolin stock 3 µl of GGF added. |
| Trypsin/EDTA 0.05%/0.02% | Gibco, Cat: 45300-019 |

### Human Schwann cell culture

### Nerve collection

Peripheral nerve samples are taken from small nerve branches from the sural nerve of the leg. Under sterile conditions, the samples are immediately collected in transfer medium (OptiMEM) and kept at room temperature for no longer than 24 hours while transferred to the laboratory. On arrival, the nerve samples are washed twice with DMEM.

Using an operating microscope, the perineurium is removed and the nerve branches are divided into fascicles and cut into 1mm pieces. The pieces are washed with DMEM/ HEPES, put in a 25cm² culture flask and covered with minimal volume human SC growth medium such that the segments remained attached to the flask rather than floating. The culture flask has to be coated with both poly-D-lysine and human laminin. Nerve pieces are incubated with SC culture medium at 37°C for 10 days, with medium changes every 2 days. At the end of the incubation period, the segments are gently removed.

### Digestion and Purification

*Day 1* The pre-digestion incubation encourage fibroblast migration out of the nerve segments as well as SC mitosis within the nerve. The nerve segments are then digested in a universal container with 2ml of cell culture medium plus 125U/ml of collagenase I and 0.8U/ml of dispase I. The container is kept at 37°C in a flask shaker to allow gentle mixing for 2 hours. If the pieces have not broken down, thus not yet digested, the process is continued for further 30-60 minutes. The resultant mixture is triturated through decreasing calibre needles (19G, 21G and 23G) filtered through a 70µm cell filter and the filter is flushed with 5ml of DMEM. The cell suspension is centrifuged at 800rpm and the pellet re-suspended in 10ml of OptiMEM and plated on a PDL/laminin double coated flask. After 24 hours, most SC have settled and the medium is changed to a human SC growth medium.
   Unlike rat SC, human SC cultures do not routinely undergo immuno-purification as the pre-digestion incubation eliminate most of the fibroblasts. However, anti human Thy 1.1 antibodies together with rabbit complement, are sometimes used to eliminate excess fibroblast growth.
*Day 2* The culture flasks are checked for cell attachment and infection. The medium is carefully taken off and the cells are gently washed with DMEM/ HEPES. After washing, 5ml of growth medium containing 10µM cytotoxic agent cytosine-β-D-arabinofuranoside (Arac C) is added and the flasks are returned to the incubator.
*Day 3* The cultures are checked for fibroblast overgrowth and incubation with fresh medium containing with Ara-C is repeated.
*Day 4* The old medium is aspirated, followed by 4 gentle washes of cells with DMEM/HEPES. 5ml of SC growth medium is added to the culture and the flasks are incubated until the SC are confluent, which takes up to 4 days.
*Day 5-8: Fibroblast depletion:* This stage is undertaken once SC (mixed with fibroblasts) are sub-confluent. SC are lifted off the flask by adding 0.25% trypsin/ EDTA (2ml for a 25cm² flask, 3-4ml for a 75cm² flask). After approximately 5 minutes incubation the cells start to lift off, then the flask is sharply tapped to detach completely all the cells. Following a check under the inverted microscope to ensure complete cell detachment, 5ml of cell growth medium is added to stop the trypsin action. The suspension is centrifuged at 800rmp for 5 minutes. The supernatant is aspirated and the cells are washed again and centrifuged leaving a residual of around 0.1ml. The cells are re-suspended and 500µl of diluted mouse anti rat Thy 1.1 antibody (dil. 1:1000 in DMEM) is added and the mixture is incubated for 10 minutes. 250µl of complement is added and the cell suspension is incubated for further 30 minutes, with occasional mixing. 10ml of cell growth medium is added to the cell suspension and centrifuged at 800rmp for 5 minutes. The cell pellet is re-suspended in 5ml of SC growth medium and incubated in a PDL/laminin coated 25cm² flask. This procedure eliminates most of the fibroblast contamination. However, on occasion the procedure is repeated if fibroblast contamination became a problem when SC are passaged. Contamination could easily be detected due to the different morphology of the cells. SC are bipolar cells with long processes, while fibroblasts show larger cell bodies with short processes.

When the SC are confluent and ready to split, they assume a characteristic swirl pattern. The medium is aspirated and the cells are lifted off by addition of 0.25% trypsin EDTA. The suspension is transferred to a universal container and centrifuged at 800rmp for 5 minutes.

### Culturing for transplantation

The SC are washed with DMEM/ HEPES, counted with a haemocytometer and plated on a PDL/laminin double coated flask with SC growth medium at density of 5x10⁵ cells/ 25cm².

Human SC grow more slowly than neonatal rat SC and need medium change every day.

To achieve an appropriate cell number for transplantation culturing has to continue for > 2 weeks after purification.

### Neonatal Schwann cell culture

Sciatic nerves from 20 neonatal Lewis rats were harvested and digested with 1% collagenase I (Worthington Biochemicals) and 0.25% trypsin (Gibco) over 45 minutes. The digestant was triturated through 21G and 23G needles and filtered through a 70 µm cell filter (Falcon). The filterant was then centrifuged at 800rpm for 5 minutes and cells were resuspended in basic medium: Dulbecco's Minimum Eagles Medium plus Glutamax®, DMEM (Gibco), penicillin, 100 iu/ml and streptomycin 100 g/ml, (Gibco), 10% fetal calf serum, FCS (Imperial Laboratories) and plated on a 25 cm² poly-D-lysine (Sigma) coated flask and kept at 37°C, 95% humidity, 5% CO₂. The following day the medium was changed to basic medium containing 10µM cytosine-β-D-arabinofuranoside (Sigma) and incubated for 38 hours to stop fibroblast growth. The majority of the cells remaining were SC and the medium was changed to SC growth medium i.e. basic medium with the addition of recombinant glial growth factor II 63ng/ml (Max^{1/2} activity, 4.8µg/ml) (Cambridge NeuroScience), and 10µM forskolin (Calbiochem). Once the cells became confluent the final stage of purification was carried out, cells were trypsinised (Gibco) and the suspension was centrifuged at 800rpm for 5 minutes. Cells were resuspended in 1 ml of medium containing mouse anti Thy 1 (di. 1:1000, Serotec, MCA04) incubated at 37°C for 30 minutes, followed by addition of 250µl of rabbit anti mouse complement (Cedarlane) and further incubation of. 15 minutes. The cells (around 120x10⁵) were then washed and grown on poly-D-lysine (Sigma) coated flasks, the medium was changed every 48 hours and SC were split; 1 in 3, on confluence (12,13). At the second passage, following purification, the rats SC were transduced.

### Genetic labelling of Schwann cells

The retroviral vector pMFG *lacZ*nls (14) was utilised to introduce modified E. *coli lacZ* marker gene that encodes the β-galactosidase protein and nuclear localisation sequence (nls) into SC. For this purpose the Moloney Murine Leukaemia Virus (MMLV) packaging cell line PT67 (Clontech, USA) was used. This contains the three retroviral structural genes in its genome but lacks the packaging signal which was provided in trans as part of the MFG *lacZ* nls containing the signal and two long terminal repeats (LTR) with *lacZ* gene cloned between them. In this way retrovirus encoding the *lacZ* gene could transduce SC, but the transduced SC could not produce any further retrovirus due to the absence of the retroviral structural genes from their genome. A stable PT67 *lacZ* nls producing clone (titre approximately 10⁶/ml) was isolated and used throughout this study.

Ten ml of growth medium (high glucose DMEM/ FCS) was put on a confluent layer of PT67 *lacZ* nls producers and kept overnight at 32°C. After incubation the medium containing retroviral particles was filtered through a 0.45µm filter (Nalgene) and added to a flask of 70% confluent SC together with the polyanion polybrene (Sigma) at 8µg/ml. The transducing medium was left on SC for 4 hours at 32°C, the medium was then taken off and SC growth medium added (15). The SC culture flask was kept at 37°C overnight and this cycle was repeated three times consecutively over 3 days. Transduction rate was 83% prior to transplantation as assessed by 5-bromo-4-chloror-3-indoyl-β-D galactosidase (X-gal) staining to detect β-galactosidase expression (16).

### Implant preparation

### Schwann cell suspension

Transduced SC were expanded in culture (no more than 7 passages) and a stock of concentrated SC suspension (160x10⁶/ml) in fibronectin (Sigma) was prepared and kept at 4°C prior to use, for no longer than 30 minutes.

### Alginate matrix and conduit filling

A sterile 4% stock solution of ultrapure, low viscosity mannuronic acid alginate (Pronova, Norway) in 0.9% saline solution was prepared. Final matrix was prepared by mixing the stock alginate (50:50 v/v) with fibronectin or SC suspension in fibronectin in.order to obtain a final 80x10⁶/ml concentration of SC. PHB conduits (Astra Tech, Sweden) were carefully filled with 30µl of alginate matrix and alginate was set in 0.1M CaCl₂ for 2 minutes and filled, conduits were gently rinsed twice in DMEM. Conduits were kept in DMEM at 4°C for no longer than 2 hours prior to implantation.

### Nerve repair model

Using an operating microscope (Zeiss, Germany), PHB conduits containing SC from Lewis rat origin were grafted in adult male Lewis (for syngeneic) or Dark Agouti (for allogencic) rats (Harlan), average weight of 180g, to bridge a gap of 1 cm in the left sciatic nerve. These two strains of rats have differences in their major histocompatibility complexes (MHC) (17). Conduits without SC were also implanted in a separate group of animals and served as control. Animals were sacrificed as 2, 3 or 6 weeks (n=6 each group) and the conduits were harvested, fixed in Zarnboni's fixative and then rinsed in 0.01 M PBS containing 15% (w/v) sucrose and 0,1 % (w/v) sodium azide and kept at 4°C.

### Tissue process and analyses

The specimens were blocked in OCT compound (Tissue-tek, Sakura, Japan), placing a piece of rat liver next to the proximal end of the nerve to identify the orientation of each sample. The specimens were sectioned longitudinally, 15µm thick, using a cryostat (Bright). Chemical X-gal staining and then fluorescent immunohistochemistry were used to examine labelled SC and regeneration and immunological parameters.

### Axonal regeneration and Schwann cell ingrowth

Axonal regeneration distance, the amount of new axonal growth as well as that of SC ingrowth into the conduits were quantified after double immunostaining on the same section. After blocking with 1% normal rat and goat sera, the sections were stained using a combination of antisera to S100 for SC identification. (dil 1:1000, rabbit polyclonal, Dako) and pan-neurofilaments (PanNF) for regenerating axons (dil 1:1000, mouse monoclonal, Affinit). Sections were incubated for 2 hours at room temperature, then washed twice with PBS (5 minutes each wash). The secondary fluorescence conjugated antibodies (dil, 1:100, goat anti-rabbit FITC conjugated and goat anti-mouse Cy3 conjugated) were added and the sections incubated for another 1 hour at room temperature. The sections were then washed with PBS (3X5min.) and mounted with Vectorshield fluorescent mountant (Vector Labs Inc,). Axonal regeneration distance was measured from the proximal stump into the graft, using a calibrated microscope graticule (mean of 3 non-consecutive sections per animal). The area, of immunostaining was taken as a measure of the quantity or the axonal regeneration and SC ingrowth into the conduits and was measured across a fixed point to allow comparison between groups, at 3 mm the proximal edge of the conduits and expressed as the percentage of immunostaining per field. A band of images were taken across the whole of the conduit and the overlapping images edited to avoid duplicate measurement..The images were captured with a SPOT digital camera (Diagnostic Images Inc) and analysed using a PC based image analysis software (Image Pro Plus, version 4, Media Cybernetics, USA).

### Transplanted Schwann cell

X-gal staining was performed on the sections to assess the presence of transduced SC. Sections were examined and scored semi-quantitatively as high, medium, low or zero. In order to assess the involvement of transplanted SC in the regeneration process, chemical X-gal staining was carried out on the same section as immunostaining for PanNF and S100, as above and the sections were examined using a combination of transmitted light and epifluorescent microscopy.

### Schwann cell characterisation

After blocking with 1% normal rat and goat sera, the following antisera were used for immunostaining on separate but consecutive sections: myelin basic protein (MBP, marker of myelinating SC, mouse monoclonal dil, 1:500, Boehringer), p75 (marker of de-differentiated SC mouse monoclonal dil. 1:40, Boehringer) and neural cell adhesion molecule (NCAM, marker of un-myelinating SC rabbit polyclonal, dil. 1:700, Chemicon). Sections were incubated at 4°C overnight and, then washed twice with PBS (5 minutes each). The secondary fluorescence conjugated antibodies (dil. 1:100, goat anti-rabbit FITC conjugated and goat anti-mouse Cy3 conjugated) were added and the sections incubated for another 1 hour at room temperature. The sections were then washed with PBS (3X5 min.) and mounted with Vectorshield fluorescent mountant (Vector Labs Inc.). The percentage area of immunostaining in a fixed area was taken as a measure of expression of each SC phenotypic markers. For each section, a digital image was taken at a fixed point, 3 mm from the proximal nerve end and equidistant from the walls of conduit. The percentage area of immunostaining was measured for each field using a PC based image analysis software as above section (mean of 3 non-consecutive sections per animal and per staining).

### Quantification of MHC class I and II

Immunostaining was carried out as above, with overnight incubation of the sections using the following antisera. MHC I (mouse monoclonal dil 1:20 Serotec, MCA51G, clone OX-18); MHC II (mouse monoclonal dil 1:20 Serotec, MCA46A anti ]-A, clone MRC OX-6). Following immunostaining sections were examined without delay and the intensity of fluorescence was taken as measure of the level of expression of MHC I and II (2 random fields were examined per section and 3 non-consecutive sections per animal and per staining). Digital images were taken at fixed settings to give comparable intensity measurements. Intensity of the field was measured using Image Pro Plus software, the calibration curve was a default straight line setting, which was confirmed with fluorescent beads of standardised luminescence.

### Lymphocyte and macrophage count

In order to assess the immune response to the conduits the number of B-lymphocytes, T-lymphocytes and macrophages were counted following immunostaining. The following antisera were used with overnight incubation at 4°C: CD2 (for B-lumphocytes, mouse monoclonal dil, 1:30 Serotec), CD45R (for T-lymphocytes mouse monoclonal dil. 1:60, Serotec) and macrophage (mouse monoclonal dil. 1:200, Serotec). Immunostaining for each antibodies was done on separate but consecutive, sections to allow comparison between the different primary antisera (3 sections per sample and antibody). The number of positively stained cells were manually counted across the whole conduits and expressed as the mean number of stained cells per section.

### Statistical analysis

One way analysis of variance (ANOVA) was performed to assess significant difference between groups, Tukey's test for comparisons between experimental and control groups, using a SigmaStat statistical analysis package (Jandel Corp, USA).

### Summary of results

An identifiable and pure population of cultured Schwann cells (SC) was obtained. Transduction of *lacZ* genetic label was carried out and a stable population of genetically modified SC was obtained. Transduced SC properties and *lacZ* expression were preserved *in vitro* for 6 months of continuous culture.

Suspension matrix is required for SC transplantation and the suitability of alginate hydrogel was confirmed by *in vitro* tests to support SC proliferation and neurite sprouting in a neuron-glial co-culture. Defects in the rat sciatic nerve injury was bridged using resorbable conduits containing SC. The results showed that the optimal number of SC required to enhance axonal regeneration was 80x10⁶/ml and alginate together with SC further improved regeneration. Following transplantation of *syngeneic* and *allogeneic* SC both improved axonal regeneration distance, but the quantity of regeneration was better and more sustained with *syngeneic* SC.

### References:

1. Liss, A.G., af Ekenstam, F.W., and Wiberg, M., Cell loss in sensory ganglia after peripheral nerve injury. An anatomical tracer study using lectin-coupled horseradish peroxidase in cats. *Scand J Plastic, Reconstr and Hand Surgery 28: 177-187, 1994.*
2. Liss, A.G., af Ekenstam, F.W., and Wiberg, M., Changes of the spinal terminal pattern of the superficial radial nerve after peripheral nerve injury. An anatomical study in cats. *Scand J Plastic, Reconstr and Hand Surgery 29: 117-131, 1995 a.*
3. Liss, A.G., af Ekenstam, F.W., and Wiberg, M., Reorganization of primary afferent nerve terminals in the brainstem after peripheral nerve injury. An anatomical study in cats. *Scand J Plastic, Reconstr and Hand Surgery 29: 185-197, 1995 b.*
4. Liss, A.G., af Ekenstam, F.W., and Wiberg, M., Loss of neurons in the dorsal root ganglia after transection of a sensory peripheral nerve. An anatomical study in monkeys. *Scand J Plastic, Reconstr and Hand Surgery 30: 1 - 6, 1996.*
5. Liss, A.G. and Wiberg, M., Primary afferent nerve endings are lost in the brainstem after peripheral nerve transection. An anatomical study in monkeys. *Anat Embryol, 196: 279 - 289, 1997 a.*
6. Liss, A.G. and Wiberg, M., Loss of nerve endings in the dorsal horn of the spinal cord after a sensory peripheral nerve injury. An anatomical study in monkeys. *Eur J of Neurosci, 9: 2187 - 2192, 1997 b.*
7. Wiberg, M., Vedung, S. and Stålberg, E., Neuronal loss after transection of the facial nerve. A morphological and neurophysiological study in the monkey. *Scand J Plastic, Reconstr and Hand Surg, 35: 135-140, 2001.*
8. Ma, J., Novikov, L., Kellerth, J-O., and Wiberg, M., Early nerve repair after postganglionic plexus injury. An experimental study of sensory and motor neuronal survival in adult rats. *Scand J Plastic, Reconstr and Hand Surg, 2001. In press.*
9. Hart, A., Brannstrom, T., Wiberg, M., and Terenghi, G., Timecourse of primary sensory neuron and satellite cell death after peripheral axotomy in the adult rat. *Exp Brain Res 2001*. *In press.*
10. Wiberg, M., Hazari, A., Kvast-Rabben, O., Ljungberg, C., Nordh, E., Pettersson, K and Terenghi, G., Sensory recovery after handreplantation. A clinical, morphological and neurophysiological investigation. *Scand J Plastic, Reconstr and Hand Surg, 2001. In press*
11. Ljungberg, C., Novikov, L., Kellerth, J-O., Ebendal, T. and Wiberg, M., The neurotrophins NGF and NT-3 reduce sensory neuronal loss in adult rats after peripheral nerve lesion. *Neurosci Letters, 262: 29 - 32, 1999.*
12. Brockes JP, Fields KL, Raff MC. Studies on cultured rat Schwann cells. I. Establishment of purified populations from cultures of peripheral nerve. Brain Res. 1979; 165: 105-118.
13. Brockes JP. Glial growth factor--a new component of the brain and pituitary. Birth Defects Orig.Artic.Ser. 1983; 19: 277-285.
14. Ferry N, Duplessis O, Houssin D, Danos O, Heard JM. Retroviral-mediated gene transfer into hepatocytes in vivo. Proc.NatLAcad.Sci.U.S.A. 1991; 88: 8377-8381.
15. Kotani H, Newton PB, Zhang S *et al*. Improved methods of retroviral vector transduction and production for gene therapy. Hum.Gene Ther. 1994; 5: 19-28.
16. Mosahebi A, Woodward B, Wiberg M, Martin R, Terenghi G. Retroviral labelling of Schwann cells: in vitro characterisation and in vivo transplantation to improve peripheral nerve regeneration. Glia 2001; 34: 8-17.
17. Günther E, Stark O. The major histocompatibility system of the rat (AgB or H-1 system). In: Goetz D, editor. The histocompatibility system in man and animals. New York: Springer, 1977: 206.

## Claims

1. Nerve repair unit comprising a resorbable polymeric support and an alginate matrix containing human Schwann cells.

2. Nerve repair unit according to claim 1, wherein the polymer of the resorbable polymeric support is selected from the group consisting of polyhydroxybutyric acid, polyglycolic acid and polylactic acid.

3. Nerve repair unit according to claim 1, wherein the alginate matrix is an ultrapure, low viscosity mannuronic acid alginate.

4. Nerve repair unit according to claim 1, wherein the resorbable polymeric support is a polyhydroxybutyric acid conduit.

5. Method of producing a nerve repair unit comprising a resorbable polymeric support and an alginate matrix containing human Schwann cells, comprising the steps of mixing an alginate in isotonic saline solution with a human Schwann cell suspension in fibronectin, supplying the mixture to the resorbable polymeric support, setting the alginate matrix with a solution of calcium chloride in human cell culture medium, rinsing with the human cell culture medium and keeping the nerve repair unit in the human cell culture medium until use.

6. Method of producing a nerve repair unit according to claim 5, wherein the polymer of the resorbable polymeric support is selected from the group consisting of polyhydroxybutyric acid, polyglycolic acid and polylactic acid, the alginate is ultrapure, low viscosity mannuronic acid alginate and the human cell culture medium is Dublbecco's Minimum Eagles Medium plus Glutamax® (DMEM).

7. Method of producing a nerve repair unit according to claim 5, wherein the human Schwann cells are produced by transporting a sample of a peripheral nerve of a human patient in a transfer medium at ambient temperature within 24 hours to a competent laboratory, upon arrival washing the sample with a human culture medium, removing the perineurum, dividing the nerve branches into fascicles and cutting them into pieces, washing the pieces with a human culture medium and placing them into a culture flask coated with both poly-D-lysine and human laminin and covering them with a Schwann cell culture medium, followed by incubation at 37°C for up to 10 days, changing the medium every 2 days, and removing the nerve segments for digestion in a container with cell culture medium plus collagenase I and dispase I until the segments have broken down, followed by trituration, filtration, washing and centrifugation, resuspending the resulting pellet in transfer medium and plating the suspension on a culture flask coated with both poly-D-lysine and human laminin and after 24 hours changing the medium to a Schwann cell culture medium, followed by cultivation for at least 7 days under conditions removing possible fibroblast contamination and changes of culture medium until the Schwann cells are confluent and ready to split, aspirating the medium and suspending the cells in a trypsinized solvent, followed by centrifugation and washing of the pellet, counting the Schwann cells with a haematocytometer and plating them on a culture flask coated with both poly-D-lysine and human laminin and covering them with a Schwann cell culture medium at a density of 5x 10⁵ cells/ 25 cm², culturing the cells for at least 14 days under change of medium every day to produce an appropriate cell number for transplantation to the patient.

## Patentansprüche

1. Nervenreparatureinheit mit einem resorbierbaren polymeren Träger und einer humane Schwannzellen enthaltenden Alginatmatrix.

2. Nervenreparatureinheit nach Anspruch 1, worin das Polymer des resorbierbaren polymeren Trägers aus der Gruppe bestehend aus Polyhydroxybuttersäure, Polyglycolsäure und Polymilchsäure ausgewählt ist.

3. Nervenreparatureinheit nach Anspruch 1, worin die Alginatmatrix ein hochreines Mannuronsäure-Alginat niedriger Viskosität ist.

4. Nervenreparatureinheit nach Anspruch 1, worin der resorbierbare polymere Träger eine Leitung aus Polyhydroxybuttersäure ist.

5. Verfahren zur Herstellung einer Nervenreparatureinheit mit einem resorbierbaren polymeren Träger und einer humane Schwannzellen enthaltenden Alginatmatrix, enthaltend die folgenden Schritte: Vermischen eines Alginats in isotonischer Salzlösung mit einer Suspension humaner Schwannzellen in Fibronectin, Zugeben der Mischung zum resorbierbaren polymeren Träger, Verfestigen der Alginatmatrix mit einer Calciumchloridlösung in einem Humanzellkulturmedium und Aufbewahren der Nervenreparatureinheit im Humanzellkulturmedium bis zur Verwendung.

6. Verfahren zur Herstellung einer Nervenreparatureinheit nach Anspruch 5, worin das Polymer des resorbierbaren polymeren Trägers aus der Gruppe bestehend aus Polyhydroxybuttersäure, Polyglycolsäure und Polymilchsäure ausgewählt ist, die Alginatmatrix hochreines Mannuronsäure-Alginat niedriger Viskosität ist und das Humanzellkulturmedium Dublebecco's Minimum Eagles Medium plus Glutamax® (DMEM) ist.

7. Verfahren zur Herstellung einer Nervenreparatureinheit nach Anspruch 5, worin die humanen Schwannzellen hergestellt werden, indem eine Probe eines peripheren Nervs eines menschlichen Patienten in einem Transfermedium bei Umgebungstemperatur innerhalb von 24 Stunden in ein geeignetes Labor gebracht wird, die Probe bei der Ankunft mit einem humanen Kulturmedium gewaschen wird, das Perineurium entfernt, die Nervenfasern in Faszikel unterteilt und in Stücke geschnitten werden, die Stücke mit einem humanen Kulturmedium gewaschen und in eine sowohl mit Poly-D-Lysin als auch mit humanem Laminin beschichtete Kulturflasche gegeben und mit einem Schwannzellen-Kulturmedium bedeckt werden, gefolgt von einer Inkubation bei 37 °C während bis zu 10 Tagen, wobei das Medium alle 2 Tage ausgewechselt wird, und die Nervensegmente zum Aufschliessen in einem Behälter mit Zellkulturmedium und Kollagenase I und Dispase I entnommen werden, bis die Segmente zerfallen sind, danach zerrieben, gefiltert, gewaschen und zentrifugiert wird, das erhaltene Pellet in Transfermedium resuspendiert und die Suspension auf eine sowohl mit Poly-D-Lysin als auch mit humanem Laminin beschichtete Kulturflasche aufgebracht wird und das Medium nach 24 Stunden durch ein Schwannzellen-Kulturmedium ersetzt wird mit nachfolgender Kultivierung während mindestens 7 Tagen unter Entfernen möglicher Verunreinigungen durch Fibroblasten und mehrfachem Auswechseln des Kulturmediums, bis die Schwannzellen konfluent und teilungsbereit sind, das Medium abgesaugt wird und die Zellen in einem trypsinisierten Lösungsmittel suspendiert werden, danach zentrifugiert und das Pellet gewaschen wird, die Schwannzellen mit einem Hämatozytometer gezählt werden und auf eine sowohl mit Poly-D-Lysin als auch mit humanem Laminin beschichtete Kulturflasche aufgebracht und mit einem Schwannzellen-Kulturmedium mit einer Dichte von 5x 10⁵ Zellen/25 cm² bedeckt werden, die Zellen während mindestens 14 Tagen mit täglichem Wechsel des Mediums kultiviert werden, um eine genügende Anzahl Zellen für die Transplantation in den Patienten zu erzeugen.

## Revendications

1. Unité de réparation nerveuse comprenant un support polymérique résorbable et une matrice d'alginate contenant des cellules Schwann humaines.

2. Unité de réparation nerveuse selon la revendication 1, où le polymère du support polymérique résorbable est choisi parmi le groupe comprenant l'acide polyhydroxybutyrique, l'acide polyglycolique et l'acide polylactique.

3. Unité de réparation nerveuse selon la revendication 1, où la matrice d'alginate est un alginate d'acide mannuronique ultrapur de basse viscosité.

4. Unité de réparation nerveuse selon la revendication 1, où le support polymérique résorbable est une voie en acide polyhydroxybutyrique.

5. Procédé de production d'une unité de réparation nerveuse comprenant un support polymérique résorbable et une matrice d'alginate contenant des cellules Schwann humaines, comprenant les étapes consistant à mélanger un alginate en solution saline isotonique avec une suspension de cellules Schwann humaines dans de la fibronectine, ajouter le mélange au support polymérique résorbable, durcir la matrice d'alginate à l'aide d'une solution de chlorure de calcium dans un milieu de culture cellulaire humain, rincer avec le milieu de culture cellulaire humain et conserver l'unité de réparation nerveuse dans le milieu de culture cellulaire humain jusqu'à l'usage.

6. Procédé de production d'une unité de réparation nerveuse selon la revendication 5, où le polymère du support polymérique résorbable est choisi parmi le groupe comprenant l'acide polyhydroxybutyrique, l'acide polyglycolique et l'acide polylactique, l'alginate est un alginate d'acide mannuronique ultrapur de basse viscosité et le milieu de culture cellulaire humain est Dublbecco's Minimum Eagles Medium plus Glutamax® (DMEM).

7. Procédé de production d'une unité de réparation nerveuse selon la revendication 5, où les cellules Schwann humaines sont produites en transportant dans un laboratoire compétent un échantillon d'un nerf périphérique d'un patient humain dans un milieu de transfert à température ambiante dans les 24 heures, en lavant à son arrivée l'échantillon à l'aide d'un milieu de culture cellulaire humain, en enlevant le périnèvre, divisant les fibres nerveuses en fascicules et en les coupant en morceaux, en lavant les morceaux à l'aide un milieu de culture cellulaire humain et en les plaçant dans un flacon de culture recouvert à la fois de poly-D-lysine et de laminine humaine et en les couvrant d'un milieu de culture à cellules Schwann, suivi d'une incubation à 37 °C pendant jusqu'à 10 jours, en échangeant le milieu tous les 2 jours, et en prélevant les segments nerveux pour leur digestion dans un récipient contenant un milieu de culture cellulaire additionné de collagénase I et de dispase I jusqu'à ce que les segments soient décomposés, suivi d'une trituration, d'une filtration, d'un lavage et d'une centrifugation, en resuspendant la pastille résultante dans un milieu de transfert et en appliquant la suspension sur un flacon de culture recouvert à la fois de poly-D-lysine et de laminine humaine et en remplaçant après 24 heures le milieu par un milieu de culture à cellules Schwann, suivi d'une culture pendant au moins 7 jours en écartant une contamination éventuelle par des fibroblastes et en échangeant plusieurs fois le milieu de culture jusqu'à ce que les cellules Schwann soient confluentes et prêtes au dédoublement, en aspirant le milieu et en suspendant les cellules dans un solvant trypsinisé, suivi par la centrifugation et du lavage de la pastille, en comptant les cellules Schwann à l'aide d'un hématocytomètre et en les appliquant sur un flacon de culture recouvert à la fois de poly-D-lysine et de laminine humaine et en les couvrant d'un milieu de culture à cellules Schwann à une densité de 5x 10⁵ cellules/25 cm², en cultivant les cellules pendant au moins 14 jours en changeant le milieu tous les jours afin de produire un nombre de cellules approprié pour la transplantation au patient.
